# EUROPEAN PATENT APPLICATION

(11) **EP 3 345 548 A1**
(43) Date of publication of application: **11.07.2018**
(21) Application number: 16841183.3
(22) Date of filing: 12.04.2016
(51) Int. Cl.: A61B 8/00, A61B 8/12

(54) **ULTRASONIC PROBE**

(30) Priority: 02.09.2015 JP 2015172884
(71) Applicant: Olympus Corporation, Hachioji-shi Tokyo 192-8507 (JP)
(72) Inventor: SAIGA, Kazuya, Hachioji-shi Tokyo 192-8507 (JP)
(74) Representative: Gunzelmann, Rainer
(86) International application number: PCT/JP2016/061807
(87) International publication number: WO 2017/038151

(57) **Abstract**

An ultrasound probe includes: a transducer unit having a plurality of ultrasound transducers; a flexible substrate 72 configured to be flat L-shaped in a relaxed state; and a plurality of coaxial wires that electrically connects the transducer unit to the flexible substrate 72. A plurality of signal lines 721S electrically connected to the transducer unit via the plurality of coaxial wires and a first return GND 721G1 that serves as a ground for the plurality of signal lines 721S are formed on the flexible substrate 72. The first return GND 721G1 is formed on an inner part 72I of the L-shaped flexible substrate 72 with respect to the plurality of signal lines 721S.

## Description

### Field

The present invention relates to an ultrasound probe.

### Background

Conventionally, an ultrasound endoscope for observing the inside of a subject by utilizing an ultrasound probe including a plurality of ultrasound transducers has been known (for example, refer to Patent Literature 1).

An ultrasound probe that is used for the ultrasound endoscope described in Patent Literature 1 includes a transducer unit (ultrasound scanning unit block) including a plurality of ultrasound transducers, a flexible substrate, and a plurality of coaxial wires (signal wire bundle) that electrically connects the transducer unit and the flexible substrate.

In the ultrasound endoscope described in Patent Literature 1, the flexible substrate is flat L-shaped in a relaxed state.

### Citation List

### Patent Literature

Patent Literature 1: JP 2012-65862 A

### Summary

### Technical Problem

A conductor pattern formed on the flexible substrate includes a plurality of signal lines for transmitting signals that are input to and output from the plurality of ultrasound transducers and a ground line that serves as a ground for the plurality of signal lines.

For example, on the flexible substrate (flat L-shaped in the relaxed state) described in Patent Literature 1, in a case where the signal line of the conductor pattern (the plurality of signal lines and the ground line) is formed on an inner part of the L shape of the flexible substrate, the following situation may occur.

That is, if the flexible substrate is flat L-shaped in the relaxed state, an inner side of the L-shaped flexible substrate may be cut off due to external force or the like. In other words, the signal line may be disconnected if the signal line is formed on the inner side of the L-shaped flexible substrate.

The present invention has been made in view of the foregoing, and an object of the invention is to provide an ultrasound probe capable of suppressing disconnection of a signal line on a flexible substrate.

### Solution to Problem

In order to solve the above described problem and achieve the object, an ultrasound probe according to the invention includes: a transducer unit having a plurality of ultrasound transducers; a flexible substrate configured to be flat L-shaped in a relaxed state; and a plurality of coaxial wires configured to electrically connect the transducer unit to the flexible substrate. The flexible substrate has thereon: a plurality of signal lines configured to be electrically connected to the transducer unit via the plurality of coaxial wires; and a first return GND that is a ground for the plurality of signal lines and is formed on an inner side of the L-shaped flexible substrate with respect to the plurality of signal lines.

In the ultrasound probe according to the invention, the flexible substrate has thereon a second return GND that is a ground for the plurality of signal lines and is formed on an outer side of the L-shaped flexible substrate with respect to the plurality of signal lines.

In the ultrasound probe according to the invention, the flexible substrate has thereon one or more third return GNDs that are grounds for the plurality of signal lines and are uniformly arranged between the first return GND and the second return GND.

In the ultrasound probe according to the invention, the number of the plurality of signal lines is a multiple of eight.

In the ultrasound probe according to the invention, the number of the plurality of signal lines is sixteen.

### Advantageous Effects of Invention

According to an ultrasound probe of the present invention, a flexible substrate is flat L-shaped in a relaxed state. From among a plurality of signal lines and a first return GND formed on the flexible substrate, the first return GND is formed on an inner side of the L-shaped flexible substrate with respect to the plurality of signal lines.

That is, the signal lines are not formed on the inner side of the L-shaped flexible substrate which may be cut off due to external force or the like. Therefore, according to the ultrasound probe of the present invention, it is possible to suppress disconnection of the signal lines.

### Brief Description of Drawings

FIG. 1 is a schematic diagram illustrating an endoscope system according to an embodiment of the present invention.
FIG. 2 is a perspective view of an endoscope connector illustrated in FIG. 1 and viewed from a front side and an upper left side.
FIG. 3 is a perspective view of an ultrasound connector removed from the endoscope connector illustrated in FIG. 2 and viewed from the inside of an exterior housing.
FIG. 4 is a schematic view illustrating a surface of an ultrasound substrate (viewed from the inside of the exterior housing) illustrated in FIG. 3.
FIG. 5A is a schematic diagram illustrating an array of a single group of FPC connector lands provided on the ultrasound substrate illustrated in FIG. 4 and to which a single FPC connector is electrically connected.
FIG. 5B is a schematic diagram illustrating an array of a single group of contacts on the single FPC connector illustrated in FIG. 4.
FIG. 6 is a schematic view illustrating an ultrasound probe that is connected to the FPC connector illustrated in FIGS. 4 and 5B.
FIG. 7 is a schematic view illustrating a flexible substrate illustrated in FIG. 6.

### Description of Embodiments

Hereinafter, modes for carrying out the present invention (hereinafter referred to as "embodiment(s)") will be described with reference to the drawings. The present invention is not limited by the embodiments described below. The same reference signs are used to designate the same elements throughout the drawings.

### [Schematic Configuration of Endoscope System]

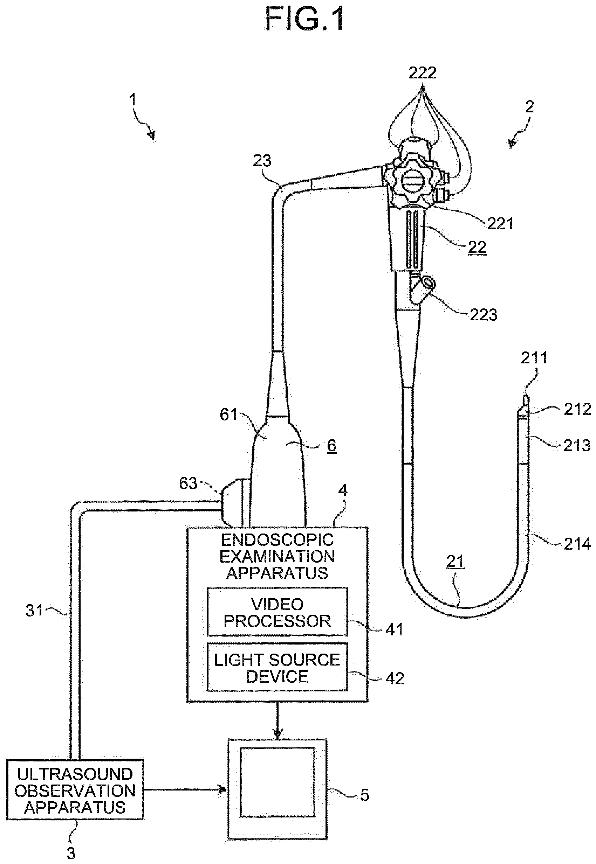

FIG. 1 is a diagram schematically illustrating an endoscope system 1 according to an embodiment of the present invention.

The endoscope system 1 is a system for performing an ultrasound diagnosis on the inside of a subject such as a human using an ultrasound endoscope. The endoscope system 1 includes, as illustrated in FIG. 1, an endoscope 2, an ultrasound observation apparatus 3, an endoscopic examination apparatus 4, and a display device 5.

The endoscope 2, a part of which is configured to be inserted into the subject, is an ultrasound endoscope having a function of sending an ultrasound pulse to a body wall in the subject and receiving an ultrasound echo reflected from the subject to output an echo signal, and a function of capturing the inside of the subject to output an image signal.

A detailed configuration of the endoscope 2 will be described later.

The ultrasound observation apparatus 3 is electrically connected to the endoscope 2 via an ultrasound cable 31 (FIG. 1), and outputs a pulse signal to the endoscope 2 via the ultrasound cable 31. The echo signal is input from the endoscope 2 to the ultrasound observation apparatus 3. The ultrasound observation apparatus 3 then performs a predetermined process on the echo signal to generate an ultrasound image.

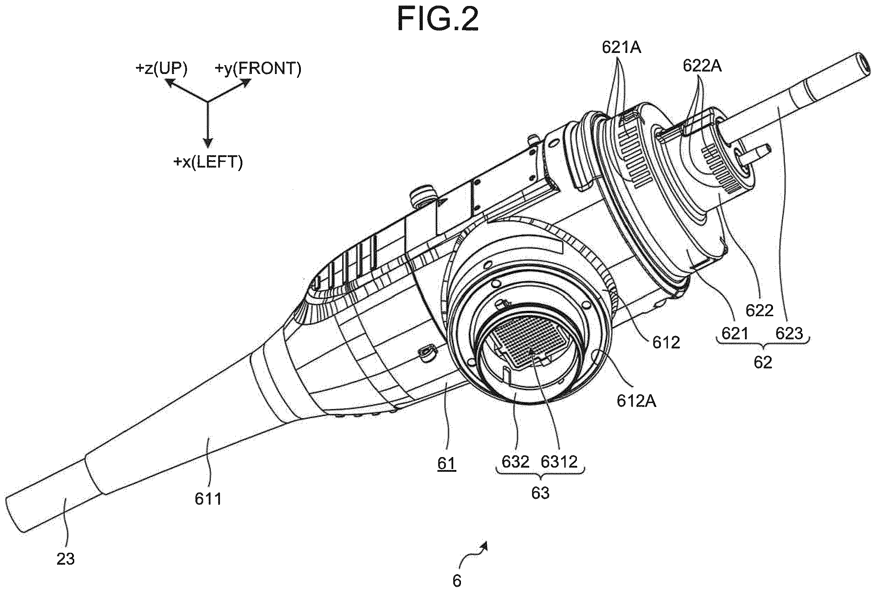

An endoscope connector 6 (refer to FIG. 2) of the endoscope 2 to be described later is detachably connected to the endoscopic examination apparatus 4. The endoscopic examination apparatus 4 includes, as illustrated in FIG. 1, a video processor 41 and a light source device 42.

The video processor 41 outputs a control signal to the endoscope 2 via the endoscope connector 6. The image signal from the endoscope 2 is input to the video processor 41 via the endoscope connector 6. The video processor 41 then performs a predetermined process on the image signal to generate an endoscope image.

The light source device 42 supplies illumination light that illuminates the inside of the subject to the endoscope 2 via the endoscope connector 6.

The display device 5 is configured by use of liquid crystal or organic electro luminescence (EL), and displays, for example, the ultrasound image generated in the ultrasound observation apparatus 3 or the endoscope image generated in the endoscopic examination apparatus 4.

### [Configuration of Endoscope]

The endoscope 2 includes, as illustrated in FIG. 1, an insertion portion 21, an operating unit 22, a universal cable 23, and the endoscope connector 6.

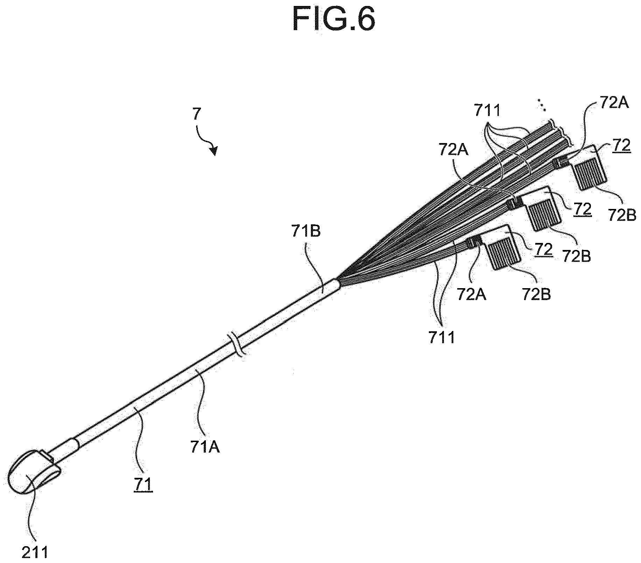

Inside the endoscope 2 (inside of the insertion portion 21, the operating unit 22, the universal cable 23, and the endoscope connector 6), a light guide for transmitting the illumination light supplied from the light source device 42, a US cable 71 (refer to FIG. 6) for the ultrasound observation (for the transmission of the pulse signal or the echo signal), and an imaging cable for the endoscope examination (for the transmission of the control signal or the image signal) are provided. The light guide, the US cable 71, and the imaging cable are not specifically illustrated.

The insertion portion 21 is a part that is inserted into the subject. The insertion portion 21 includes, as illustrated in FIG. 1, a transducer unit 211, a rigid member 212, a bending portion 213, and a flexible pipe portion 214. The transducer unit 211 is provided at a distal end. The rigid member 212 is joined to a proximal end side (close to the operating unit 22) of the transducer unit 211. The bending portion 213 is joined to a proximal end side of the rigid member 212 and is bendable. The flexible pipe portion 214 is joined to a proximal end side of the bending portion 213 and has flexibility.

Inside the insertion portion 21 (the rigid member 212, the bending portion 213, and the flexible pipe portion 214), an image guide for guiding an optical image of the inside of the subject and a treatment tool tube into which various treatment tools (for example, a puncture needle or the like) are inserted are provided in addition to the light guide and the US cable 71 (refer to FIG. 6) mentioned above. The image guide and the treatment tool tube are not specifically illustrated.

In the example illustrated in FIG. 1, the transducer unit 211 is a convex ultrasound probe and configured in such a manner that a plurality of ultrasound transducers (not illustrated) is regularly arrayed so as to form a projecting arc.

The ultrasound transducer as used herein has an acoustic lens, a piezoelectric element, and a matching layer, and acquires the ultrasound echo that contributes to an ultrasound tomographic image of the inside of the body wall in the subject. In the embodiment, the number of ultrasound transducers is a multiple of eight.

The transducer unit 211 then converts, into the ultrasound pulse, the pulse signal input from the ultrasound observation apparatus 3 via the above-mentioned US cable 71 (refer to FIG. 6) and the ultrasound cable 31, and sends the ultrasound pulse to the inside of the subject. The transducer unit 211 also converts the ultrasound echo reflected from the inside of the subject into the electrical echo signal, and outputs the electrical echo signal to the ultrasound observation apparatus 3 via the above US cable 71 (refer to FIG. 6) and the ultrasound cable 31.

The rigid member 212 is a hard member including a resin material and has a substantially columnar shape.

In the rigid member 212, an observation window, an illumination window, and a treatment tool passage or the like, which are not specifically illustrated, are formed.

The observation window, the illumination window, and the treatment tool passage are holes passing through the rigid member 212 from a proximal end (end portion close to the operating unit 22) to a distal end, and specifically have the following functions.

The observation window is the hole for acquiring the optical image of the inside of the subject. An incident end side of the above-mentioned image guide is inserted into the observation window. An objective lens (not illustrated) is linked to the incident end of the above-mentioned image guide.

The illumination window is the hole for illuminating the inside of the subject with the illumination light. An emitting end side of the above-mentioned light guide is inserted into the illumination window.

The treatment tool passage is the hole for causing various treatment tools to protrude to the outside. The above-mentioned treatment tool tube is connected to the treatment tool passage.

The operating unit 22 is a part that is joined to a proximal end side of the insertion portion 21 and accepts various operations from a medical doctor or the like. The operating unit 22 includes, as illustrated in FIG. 1, a bending knob 221 for performing the curve operation on the bending portion 213 and a plurality of operating members 222 for performing various operations.

A treatment tool insertion opening 223 communicating with the above-mentioned treatment tool tube and configured to cause various treatment tools to be inserted into the treatment tool tube is formed in the operating unit 22.

Furthermore, an imaging sensor (not illustrated) and an optical system (not illustrated) are arranged inside the operating unit 22. The imaging sensor outputs the image signal that depends on the optical image of the inside of the subject. The optical system forms the optical image guided by the above-mentioned image guide on the imaging sensor. The image signal output from the imaging sensor is transmitted to the endoscopic examination apparatus 4 (video processor 41) via the above-mentioned imaging cable.

The universal cable 23, one end of which is connected to the operating unit 22, is a cable in which the light guide, the US cable 71 (refer to FIG. 6), and the imaging cable or the like mentioned above are incorporated.

The endoscope connector 6 is a connector provided at the other end of the universal cable 23 and configured to be connected to the ultrasound cable 31 connected to the ultrasound observation apparatus 3 and the endoscopic examination apparatus 4 (the video processor 41 and the light source device 42).

### [Configuration of Endoscope Connector]

Next, a configuration of the endoscope connector 6 will be described.

Hereinafter, with reference to a posture in which the endoscope connector 6 is connected to the endoscopic examination apparatus 4, an upper side in the posture is referred to as "up", a lower side in the posture is referred to as "down", a side close to the endoscopic examination apparatus 4 is referred to as "front", a side apart from the endoscopic examination apparatus 4 is referred to as "rear", a left side viewed from the front side in the posture is referred to as "left", and a right side viewed from the front side in the posture is referred to as "right".

FIG. 2 is a perspective view of the endoscope connector 6 viewed from the front side and the upper left side.

In FIG. 2, XYZ orthogonal coordinates are illustrated to identify the above-mentioned "up-down", "front-rear", and "right-and-left" of the endoscope connector 6. As used herein, the +Z axis direction is the "up direction" of the endoscope connector 6. The +X axis direction is the "left direction" of the endoscope connector 6. The +Y axis direction is the "front direction" of the endoscope connector 6.

The endoscope connector 6 includes, as illustrated in FIG. 2, an exterior housing 61, a plug portion 62, and an ultrasound connector 63.

The exterior housing 61 has, as illustrated in FIG. 2, a substantially cylindrical shape extending in the front-rear direction (Y axis direction). The universal cable 23 (the light guide, the US cable 71 (refer to FIG. 6), and the imaging cable or the like mentioned above) is inserted into the exterior housing 61 via an opening part on the rear side thereof. As illustrated in FIG. 2, a bending prevention member 611 is provided on the rear side of the exterior housing 61.

As illustrated in FIG. 2, a bulge portion 612 bulging in the +X axis direction is formed on a side surface of the above-described exterior housing 61.

The bulge portion 612 communicates with the inside of the exterior housing 61 and has a hollow shape. In the bulge portion 612, as illustrated in FIG. 2, an opening plane is located on the YZ plane, and an attachment hole 612A communicating with the inside and outside of the exterior housing 61 is formed. The attachment hole 612A is a hole to which the ultrasound connector 63 is attached.

The plug portion 62 is a part that is inserted into the endoscopic examination apparatus 4 and connected to the video processor 41 and the light source device 42. As illustrated in FIG. 2, the plug portion 62 is attached to an opening part on the front side of the exterior housing 61. The plug portion 62 includes, as illustrated in FIG. 2, first and second electrical connector portions 621 and 622 and a light guide base 623.

As illustrated in FIG. 2, the first electrical connector portion 621 is located on the rearmost side of the plug portion 62, and has a columnar shape extending in the front-rear direction.

A plurality of first electrical contact points 621A is provided on a part of an outer peripheral surface of the first electrical connector portion 621 along a circumferential direction.

As illustrated in FIG. 2, the second electrical connector portion 622 is integrally formed on the front side of the first electrical connector portion 621, and has a columnar shape having an outer diameter dimension smaller than an outer diameter dimension of the first electrical connector portion 621.

A plurality of second electrical contact points 622A is provided on a part of an outer peripheral surface of the second electrical connector portion 622 along a circumferential direction.

The plurality of first and second electrical contact points 621A and 622A are electrically connected to the above-mentioned imaging cable. The plurality of first and second electrical contact points 621A and 622A are also electrically connected to the video processor 41, with the plug portion 62 inserted into the endoscopic examination apparatus 4. In other words, the plurality of first and second electrical contact points 621A and 622A are the parts that electrically connect the above-mentioned imaging cable to the video processor 41.

The light guide base 623 is attached to an end surface on the front side of the second electrical connector portion 622, and protrudes from the end surface on the front side in the +Y axis direction.

An incident end side of the above-mentioned light guide is inserted into the light guide base 623. The light guide base 623 is also connected to the light source device 42, with the plug portion 62 inserted into the endoscopic examination apparatus 4. In other words, the light guide base 623 is the part that optically connects the above-mentioned light guide to the light source device 42.

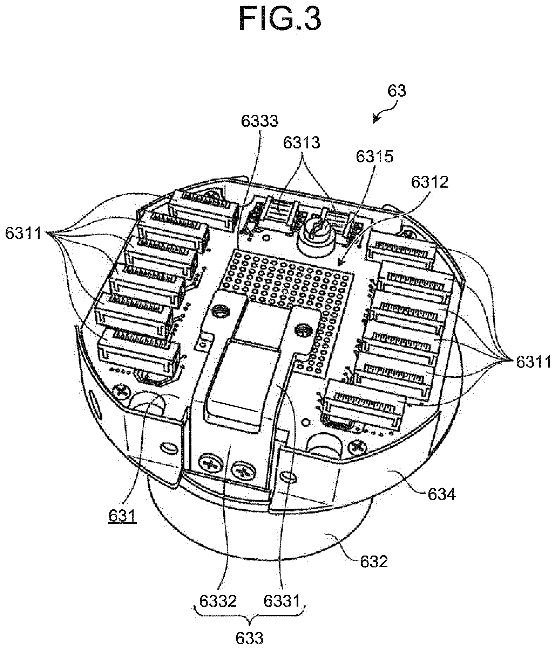

FIG. 3 is a perspective view of the ultrasound connector 63 removed from the endoscope connector 6 and viewed from the inside of the exterior housing 61.

The ultrasound connector 63 is an electrical connector for electrically connecting the above-mentioned US cable 71 (refer to FIG. 6) to the ultrasound cable 31. The ultrasound connector 63 includes, as illustrated in FIG. 2 or 3, an ultrasound substrate 631 (FIG. 3), a frame member 632, an electrical connection member 633 (FIG. 3), and a spacer 634 (FIG. 3).

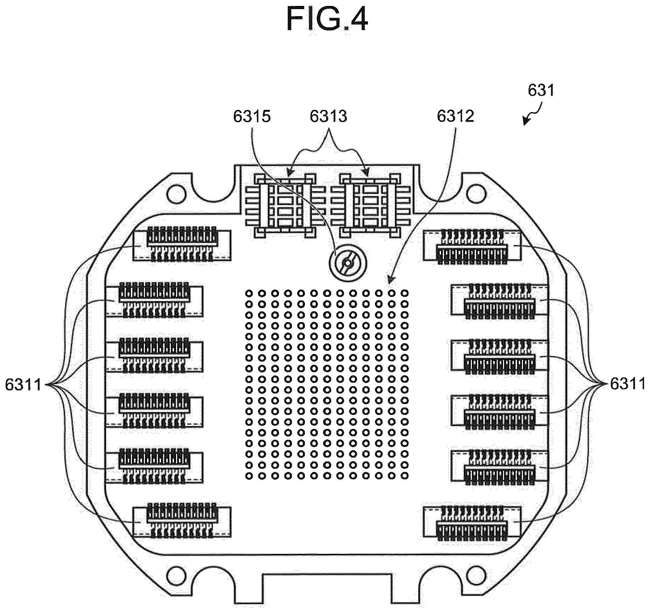

FIG. 4 is a schematic view illustrating a surface of the ultrasound substrate 631 (viewed from the inside of the exterior housing 61).

The ultrasound substrate 631 is a substrate having a substantially disk-like shape. A plurality of FPC connectors 6311 (FIGS. 3 and 4), a plurality of pin-shaped terminals 6312 (FIGS. 2 to 4), and a plurality of slide switches 6313 (FIGS. 3 and 4) are mounted on the surface of the ultrasound substrate 631.

The plurality of FPC connectors 6311 (twelve in the embodiment) is connectors to which a plurality of flexible substrates 72 (refer to FIG. 6) of an ultrasound probe 7 (refer to FIG. 6) is connected.

A configuration of the ultrasound probe 7 will be described later.

The twelve FPC connectors 6311 are separated into two groups of six and arranged on both sides (both the right and left sides in FIG. 4) with respect to the plurality of pin-shaped terminals 6312. In FIG. 4, the six FPC connectors 6311 arranged on the right side and the six FPC connectors 6311 arranged on the left side are arranged in an upside-down posture in FIG. 4.

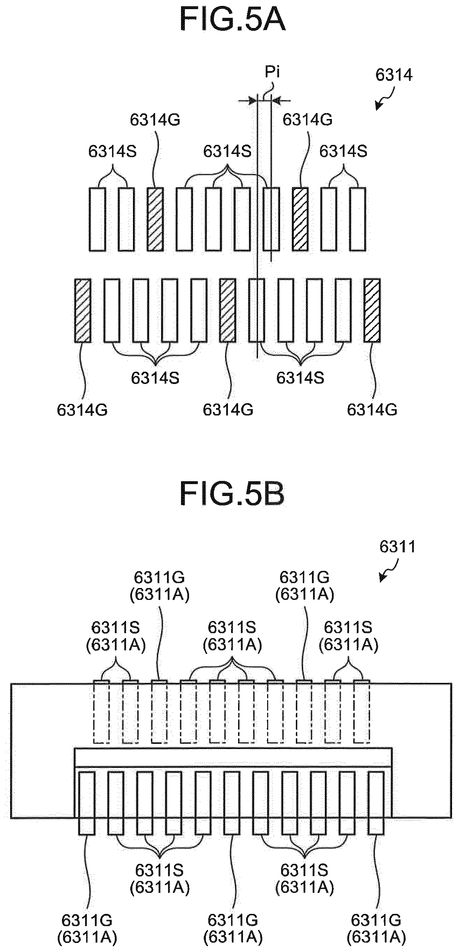

FIG. 5A is a schematic diagram illustrating an array of a single group of FPC connector lands 6314 provided on the ultrasound substrate 631 and to which a single FPC connector 6311 is electrically connected. FIG. 5B is a schematic diagram illustrating an array of a single group of contacts 6311A on the single FPC connector 6311.

The up-down direction and the right-and-left direction in FIGS. 5A and 5B are the same as the up-down direction and the right-and-left direction in FIG. 4, respectively. The single group of FPC connector lands 6314 to which the single FPC connector 6311 out of the six FPC connectors 6311 arranged on the right side in FIG. 4 is electrically connected is illustrated in FIG. 5A. Similarly, the single FPC connector 6311 out of the six FPC connectors 6311 arranged on the right side in FIG. 4 is illustrated in FIG. 5B.

On the surface of the ultrasound substrate 631, a plurality of groups of FPC connector lands 6314 (twelve groups in the embodiment) (only the single group of FPC connector lands out of the six groups of FPC connector lands provided on the right side of the ultrasound substrate 631 in FIG. 4 is illustrated in FIG. 5A) is provided.

The single group of FPC connector lands 6314 is arranged side by side in two rows that are next to each other in the up-down direction in FIG. 5A.

More specifically, a plurality of FPC connector lands 6314 (ten connector lands in the embodiment) arranged side by side in the upper first row is arranged side by side at predetermined pitches. A plurality of FPC connector lands 6314 (eleven connector lands in the embodiment) arranged side by side in the lower second row is arranged side by side at pitches similar to those of the plurality of FPC connector lands 6314 arranged side by side in the first row. When viewed from above in FIG. 5A, each of the ten FPC connector lands 6314 arranged side by side in the first row is provided at a center position of the adjacent FPC connector lands 6314 arranged side by side in the second row.

Out of the twenty one FPC connector lands 6314, five FPC connector lands 6314G in total (FIG. 5A (represented by hatching)), i.e. the third one from the left and the third one from the right in the first row in FIG. 5A and both the right and left ones and the central one in the second row in FIG. 5A, are respectively electrically connected to a plurality of ground wires (not illustrated) that is a conductor pattern provided on the ultrasound substrate 631. Sixteen FPC connector lands 6314S (FIG. 5A) other than the five FPC connector lands 6314 are respectively electrically connected to a plurality of signal wires (not illustrated) that is a conductor pattern provided on the ultrasound substrate 631.

In the embodiment, the above pitch is set to 0.6 mm. Specifically, when viewed from above in FIG. 5A, a pitch Pi between the FPC connector lands 6314 adjacently arranged side by side in the first and second rows is set to 0.3 mm.

Thus, since the pitch Pi is comparatively small, namely, 0.3 mm, the ultrasound substrate 631 can be reduced in size.

Although each of the six groups of FPC connector lands provided on the right side of the ultrasound substrate 631 in FIG. 4 is arrayed in a state illustrated in FIG. 5A, each of the six groups of FPC connector lands provided on the left side is arrayed in an upside-down state of FIG. 5A with respect to the state illustrated in FIG. 5A.

The FPC connector 6311 includes, as illustrated in FIG. 5B, twenty-one contacts 6311A arrayed in a similar way to the array of the single group of FPC connector lands 6314 illustrated in FIG. 5A. The respective twenty-one contacts 6311A are electrically connected to the single group of FPC connector lands 6314 by means of solder or the like. Specifically, out of the twenty-one contacts 6311A, five contacts 6311G (FIG. 5B) are electrically connected to the respective five FPC connector lands 6314G, and sixteen contacts 6311S (FIG. 5B) are electrically connected to the respective sixteen FPC connector lands 6314S.

As illustrated in FIG. 3 or 4, the plurality of pin-shaped terminals 6312 is arrayed in a matrix on a substantially central part of the ultrasound substrate 631. The plurality of pin-shaped terminals 6312 is electrically connected to the ultrasound probe 7 via the twelve FPC connectors 6311 and electrically connected to the ultrasound cable 31 when the ultrasound cable 31 is connected to the ultrasound connector 63.

Each of the plurality of slide switches 6313 (two switches in the embodiment) is a switch of four bits or more which generates a probe ID (for example, information indicating the type of the transducer unit 211 (type such as a convex type and a radial type or the like)) which is individual information of the ultrasound probe 7.

Specifically, since the two slide switches 6313 are provided, the probe ID of eight bits or more can be generated only by means of the on/off operation for the switch, and the convenience can be improved.

A vent, which is not specifically illustrated, is formed in the ultrasound substrate 631 so as to pass through the ultrasound substrate 631 from the front to the back. As illustrated in FIG. 3 or 4, a ventilation base 6315 having a hole communicating with the vent is attached to the vent.

A ventilation waterproof sheet, which is not specifically illustrated, having a ventilation property and a waterproof property is provided inside of the ventilation base 6315 so as to close the above-mentioned hole.

The vent and the ventilation base 6315 mentioned above are used for what is called a watertight inspection for confirming the waterproof property of the endoscope connector 6.

The watertight inspection is performed in the following manner. For example, a waterproof cap (not illustrated) is put on the ultrasound connector 63, and pressurized air from a separate pressure device is delivered to the inside of the ultrasound connector 63 (inside of the endoscope connector 6) via the waterproof cap, during which the endoscope connector 6 is submerged in water. At this time, whether air bubbles are generated or not is examined, and the waterproof property of the endoscope connector 6 is confirmed.

To sum up, since the ventilation base 6315 is provided inside the ultrasound connector 63, not inside the exterior housing 61, even when, for example, a person accidentally forgets to put the waterproof cap on the ultrasound connector 63 and performs a process of cleaning the endoscope connector 6, only the ultrasound substrate 631 or the like is submerged in water, and the submergence of various electrical members within the exterior housing 61 can be avoided.

As illustrated in FIG. 2 or 3, the frame member 632 is a part including a cylindrical metal member and mechanically connected to a connector close to the ultrasound cable 31. The frame member 632 supports the ultrasound substrate 631 at an opening part on one end side (close to the inside of the exterior housing 61).

As illustrated in FIG. 3, the electrical connection member 633 includes a metal member having an L-shaped cross-section. In such a posture that a part 6331 on one end side of the L-shaped cross-section faces the surface (close to the inside of the exterior housing 61) of the ultrasound substrate 631, a part 6332 on the other end side of the L-shaped cross-section is connected to the frame member 632. An insulation sheet 6333 is arranged between the part 6331 on the one end side of the L-shaped cross-section of the electrical connection member 633 and the surface (plurality of pin-shaped terminals 6312) of the ultrasound substrate 631.

An extension end 71B (refer to FIG. 6) of the above-mentioned US cable 71 (refer to FIG. 6) extending from the transducer unit 211 is fixed to the part 6331 on the one end side of the L-shaped cross-section of the electrical connection member 633.

As illustrated in FIG. 3, the spacer 634 is a substantially cylindrical metal member (shield member) that covers a part of an outer edge portion of the ultrasound substrate 631, and fixed to the one end side (close to the inside of the exterior housing 61) of the frame member 632.

The ultrasound connector 63 is fixed by means of a screw or the like, with a side including the ultrasound substrate 631 inserted into the attachment hole 612A.

### [Configuration of Ultrasound Probe]

Next, the configuration of the ultrasound probe 7 will be described.

FIG. 6 is a schematic view illustrating the ultrasound probe 7 that is connected to the FPC connector 6311.

The ultrasound probe 7 includes, as illustrated in FIG. 6, the transducer unit 211, the US cable 71, and the plurality of flexible substrates 72.

As mentioned above, the US cable 71 is the cable that transmits the pulse signal or the echo signal between the transducer unit 211 and the ultrasound observation apparatus 3. More specifically, as illustrated in FIG. 6, the US cable 71 is configured in such a manner that a plurality of coaxial wires 711 respectively electrically connected to the plurality of ultrasound transducers of the transducer unit 211 is bundled by means of a covering tube 71A.

As illustrated in FIG. 6, at the extension end extending from the transducer unit 211, the plurality of coaxial wires 711 is bundled for each unit (bundled for each sixteen coaxial wires in the embodiment), and the plurality of flexible substrates 72 (twelve substrates in the embodiment) is attached to the respective bundles.

In other words, the plurality of coaxial wires 711 electrically connects the transducer unit 211 to the twelve flexible substrates 72.

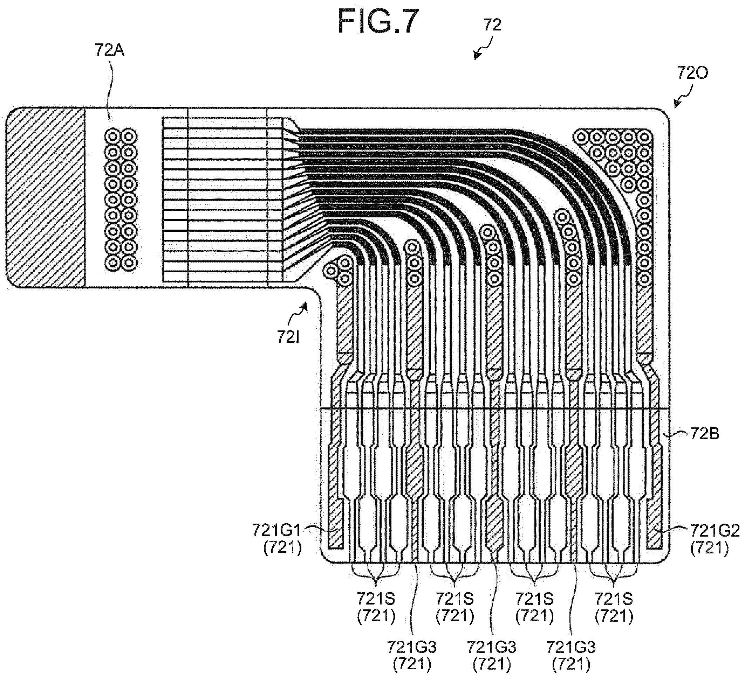

FIG. 7 is a schematic view illustrating the flexible substrate 72.

In FIG. 7, a surface of the flexible substrate 72 in a relaxed state is illustrated.

As illustrated in FIG. 6 or 7, the flexible substrate 72 is flat L-shaped extending from one end 72A to the other end 72B in a relaxed state. A single bundle of (sixteen) coaxial wires 711 out of the plurality of bundles is electrically connected to the one end 72A of the flexible substrate 72. The other end 72B of the flexible substrate 72 is connected to the FPC connector 6311.

As illustrated in FIG. 7, a conductor pattern 721 including sixteen signal lines 721S and five return GNDs 721G1 to 721G3 is formed on the flexible substrate 72.

The sixteen signal lines 721S are conductively connected to the single bundle of respective (sixteen) coaxial wires 711. The sixteen signal lines 721S are also conductively connected to the respective sixteen contacts 6311S (sixteen FPC connector lands 6314S), with the other end 72B of the flexible substrate 72 connected to the FPC connector 6311.

The five return GNDs 721G1 to 721G3 are conductively connected to the respective five contacts 6311G (five FPC connector lands 6314G), with the other end 72B of the flexible substrate 72 connected to the FPC connector 6311.

As illustrated in FIG. 7, the sixteen signal lines 721S and the five return GNDs 721G1 to 721G3 are arranged side by side between an inner part 72I and an outer part 72O of the L shape of the flexible substrate 72.

More specifically, the return GND 721G1 is formed on the inner part 72I of the L shape of the flexible substrate 72. In other words, the return GND 721G1 corresponds to a first return GND according to the present invention.

The return GND 721G2 is formed on the outer part 72O of the L shape of the flexible substrate 72. In other words, the return GND 721G2 corresponds to a second return GND according to the present invention.

The remaining three return GNDs 721G3 are uniformly allocated, with the respective four signal lines 721S sandwiched between the three return GNDs 721G3 and the adjacent other return GNDs 721G1 to 721G3. In other words, each of the three return GNDs 721G3 corresponds to a third return GND according to the present invention.

In the ultrasound probe 7 according to the embodiment, the flexible substrate 72 is flat L-shaped in the relaxed state. Out of the sixteen signal lines 721S and the five return GNDs 721G1 to 721G3 formed on the flexible substrate 72, the return GND 721G1 is formed on the inner part 72I of the L-shaped flexible substrate 72 with respect to the sixteen signal lines 721S.

In other words, the signal line 721S is not formed on the inner part 72I which might be cut off due to the external force or the like. Therefore, the ultrasound probe 7 according to the embodiment is able to suppress the disconnection of the signal line 721S.

In addition, since the four return GNDs 721G2 and 721G3 are provided in addition to the return GND 721G1, the return GND for the signal line 721S can be stably maintained by the four return GNDs 721G2 and 721G3 even when the return GND 721G1 is disconnected.

If the external force is exerted on the flexible substrate 72, the outer part 72O of the L-shaped flexible substrate 72 might also be cut off.

In this regard, in the ultrasound probe 7 according to the embodiment, out of the sixteen signal lines 721S and the five return GNDs 721G1 to 721G3 formed on the flexible substrate 72, the return GND 721G2 is formed on the outer part 72O of the L-shaped flexible substrate 72 with respect to the sixteen signal lines 721S.

In other words, the signal line 721S is not formed on the outer part 72O which might be cut off due to the external force or the like. Therefore, the ultrasound probe 7 according to the embodiment can further suppress the disconnection of the signal line 721S.

If the pulse signal or the echo signal is transmitted in the ultrasound probe 7 while a magnetic material such as plating is used for the contact point, a magnetic field occurs in the contact part due to the ultrasound vibration in the transducer unit 211, and noise (magnetostrictive noise) occurs in the ultrasound image.

In this regard, the present applicant has studied the noise and found that the noise can be reduced when the return GNDs are uniformly disposed.

In the ultrasound probe 7 according to the embodiment, the five return GNDs 721G1 to 721G3 are uniformly allocated. Therefore, the ultrasound probe 7 according to the embodiment can improve a noise resistance property by means of the GND stability and reduce the above-mentioned magnetostrictive noise.

### (Other Embodiments)

Although the embodiment for practicing the present invention has been described so far, the present invention should not be limited only by the above-mentioned embodiment.

In the above-mentioned embodiment, the endoscope system 1 has both the function for generating the ultrasound image and the function for generating the endoscope image. However, the endoscope system 1 is not limited to this example, and may be configured in such a manner that the function for generating the endoscope image is removed.

In the above-mentioned embodiment, the sixteen signal lines 721S are provided. However, a different number of signal lines 721S may be provided as long as the number is a multiple of eight. Similarly, the number of return GNDs 721G3 is also not limited to three, and may be one or a different number as long as the return GNDs 721G3 can be uniformly allocated.

In the above-mentioned embodiment, the endoscope system 1 may be used not only in the medical field but also in the industrial field, and may serve as an endoscope system for observing the inside of a subject such as a machine structure.

### Reference Signs List

1 ENDOSCOPE SYSTEM
2 ENDOSCOPE
3 ULTRASOUND OBSERVATION APPARATUS
4 ENDOSCOPIC EXAMINATION APPARATUS
5 DISPLAY DEVICE
6 ENDOSCOPE CONNECTOR
7 ULTRASOUND PROBE
21 INSERTION PORTION
22 OPERATING UNIT
23 UNIVERSAL CABLE
31 ULTRASOUND CABLE
41 VIDEO PROCESSOR
42 LIGHT SOURCE DEVICE
61 EXTERIOR HOUSING
62 PLUG PORTION
63 ULTRASOUND CONNECTOR
71 US CABLE
71A COVERING TUBE
71B EXTENSION END
72 FLEXIBLE SUBSTRATE
72A ONE END
72B OTHER END
72I INNER PART
72O OUTER PART
211 TRANSDUCER UNIT
212 RIGID MEMBER
213 BENDING PORTION
214 FLEXIBLE PIPE PORTION
221 BENDING KNOB
222 OPERATING MEMBER
223 TREATMENT TOOL INSERTION OPENING
611 BENDING PREVENTION MEMBER
612 BULGE PORTION
612A ATTACHMENT HOLE
621, 622 FIRST AND SECOND ELECTRICAL CONNECTOR PORTIONS
621A, 622A FIRST AND SECOND ELECTRICAL CONTACT POINTS
623 LIGHT GUIDE BASE
631 ULTRASOUND SUBSTRATE
632 FRAME MEMBER
633 ELECTRICAL CONNECTION MEMBER
634 SPACER
711 COAXIAL WIRE
721G1 to 721G3 RETURN GND
721S SIGNAL LINE
6311 FPC CONNECTOR
6311A, 6311G, 6311S CONTACT
6312 PIN-SHAPED TERMINAL
6313 SLIDE SWITCH
6314, 6314G, 6314S FPC CONNECTOR LAND
6315 VENTILATION BASE
6331, 6332 PART
6333 INSULATION SHEET
Pi PITCH

## Claims

1. An ultrasound probe comprising:
a transducer unit having a plurality of ultrasound transducers;
a flexible substrate configured to be flat L-shaped in a relaxed state; and
a plurality of coaxial wires configured to electrically connect the transducer unit to the flexible substrate, wherein
the flexible substrate has thereon:
a plurality of signal lines configured to be electrically connected to the transducer unit via the plurality of coaxial wires; and
a first return GND that is a ground for the plurality of signal lines and is formed on an inner side of the L-shaped flexible substrate with respect to the plurality of signal lines.

2. The ultrasound probe according to claim 1, wherein
the flexible substrate has thereon a second return GND that is a ground for the plurality of signal lines and is formed on an outer side of the L-shaped flexible substrate with respect to the plurality of signal lines.

3. The ultrasound probe according to claim 2, wherein
the flexible substrate has thereon one or more third return GNDs that are grounds for the plurality of signal lines and are uniformly arranged between the first return GND and the second return GND.

4. The ultrasound probe according to any one of claims 1 to 3, wherein
the number of the plurality of signal lines is a multiple of eight.

5. The ultrasound probe according to claim 4, wherein
the number of the plurality of signal lines is sixteen.
